(19) Europäisches Patentamt
European Patent Office
Office européen des brevets

(11) Publication number: **0 002 322**
**A2**

(12) **EUROPEAN PATENT APPLICATION**

(21) Application number: 78300595.2

(22) Date of filing: 06.11.78

(51) Int. Cl.[2]: C 07 H 21/04
//C07H1/02, C12K1/02

(30) Priority: 08.11.77 US 849606

(43) Date of publication of application:
13.06.79 Bulletin 79/12

(84) Designated contracting states:
BE CH DE FR GB NL SE LU

(71) Applicant: GENENTECH, INC.
460 Point San Bruno Boulevard
So. San Francisco California(US)

(72) Inventor: Itakura, Keiichi
1180 Coronet Avenue
Pasadena, California(US)

(74) Representative: Goldin, Douglas Michael et al,
J.A. KEMP & CO. 14, South Square Gray's Inn
London WC1R 5EU(GB)

(54) **Improved Triester process for the synthesis of oligonucleotides.**

(57) Oligonucleotides of a defined sequence, particularly oligodeoxyribonucleotides are prepared by an improved "triester" process. The oligodeoxyribonucleotides can be converted to DNA segments which can be used for insertion in plasmid DNA to code the plasmid DNA for predetermined purposes. For example, the plasmid DNA can be coded to stimulate the synthesis of polypeptide hormones by a bacterium such as *E. Coli.*

EP 0 002 322 A2

Croydon Printing Company Ltd.

0002322



## DESCRIPTION

## IMPROVED TRIESTER PROCESS FOR THE SYNTHESIS OF OLIGONUCLEOTIDES

### Field of Invention

This invention relates to oligonucleotides. In one aspect, this invention relates to synthetic deoxyribonucleic acid (DNA).

In another aspect it relates to oligodeoxyribonucleotides as precursors to DNA.

In yet another aspect it relates to procedures for synthesizing oligonucleotides of defined sequence.

More specifically, this invention relates to procedures for synthesizing oligodeoxyribonucleotides.

### Background

The ability to synthesize oligodeoxyribonucleotides having a defined sequence of the four different nucleotides that make up DNA is becoming increasingly important since it is now possible to insert relatively short DNA segments into plasmid DNA to code the plasmid DNA for a variety of purposes, e.g., to stimulate production of mammalian peptide hormones.

One successful synthetic scheme has been dubbed the "diester approach" which may be represented by the following reaction scheme to form a dimeric nucleotide:

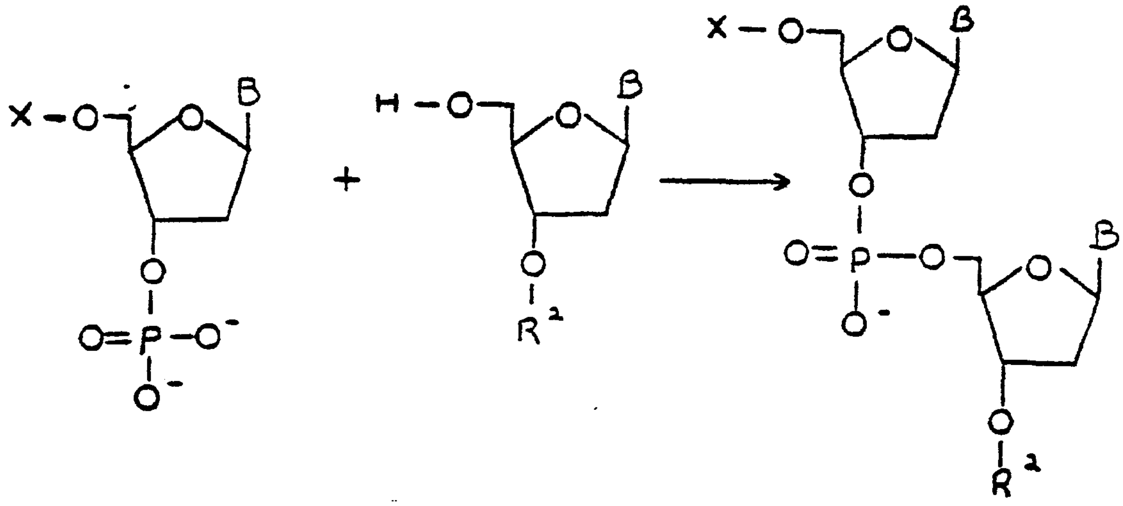

wherein B is a base group selected from thymine and protected adenine, cytosine and quanine; X is a protective group for the 5'-hydroxyl function and $R^2$ a protective group for the 3'-hydroxyl function. Subsequent couplings of the dimeric nucleotide with a mononucleotide or with larger nucleotides can be used to build longer and longer oligonucleotide chains. A judicious selection of the reactants allows the formation of chains in which the group B has the desired sequence. The "diester approach" derives its name, of course, from the fact that the coupled product is a phosphate diester.

The sequence of the B group provides the coding for DNA. Thus, it is known that each amino acid in a polypeptide has a corresponding coding in DNA made up of a specific sequence of 3 nucleotides. These groups of three nucleotides are referred to as codons.

The diester method, though effective, is critically deficient since yields drop rapidly with increasing chain length, purification procedures are time consuming and the overall scheme is difficult to scale up.

An improved procedure has displaced the diester approach and is known to the art as the "triester approach". This procedure involves the coupling of a nucleotide having a masked 3'-phosphate group with a nucleoside or nucleotide having an available 5'-hydroxyl group. This procedure may be represented by the following scheme:

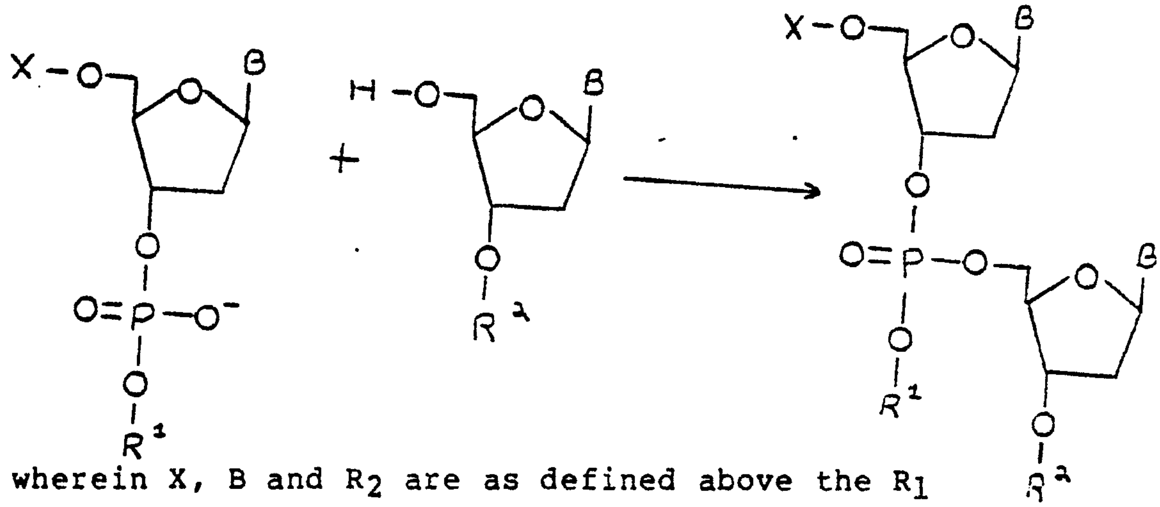

wherein X, B and $R_2$ are as defined above the $R_1$ is an organic group which functions to mask the 3'-phosphate group and which can be cleaved by base. Typically the group is a p-chlorophenyl moiety.

The "triester approach" derives its name from the fact that the coupled product is a phosphate triester. Further details of this process may be found in Itakura et al, "A Modified Triester Method For The Synthesis of Deoxyribopolynucleotides", Can J. Chem. 51, 3649 (1973); Itakura et al, "Chemical Synthesis And Sequence Studies of Deoxyribonucleotides Which Constitute The Duplex Sequence Of The Lactose Operator of E. Coli," J. Biol. Chem. 250; 4592 (1975); Bahl et al, "Chemical And Enzymatic Synthesis Of The Lactose Operator Of E. Coli And Its Binding To The Lac Repressor", Proc. Nat. Acad. Sci. USA 73, 91 (1976); and Itakura et al, "Improved Triester Approach For The Synthesis Of Pentadecathymidylic Acid," J. Am Chem. Soc., 97, 7327 (1975). The disclosures of these are incorporated by reference.

Although a great improvement over the diester approach, the triester approach is still relatively slow overall since it requires extensive chromatography to purify each block of oligodeoxyribonucleotide as it is formed. This purification is necessary to separate the coupled product from the non-reacted 5'-hydroxyl component. In some instances, a further chemical reaction and purification is necessary to obtain coupled product of sufficient purity for use in further coupling reactions.

Accordingly one object of this invention is to provide an improved chemical synthesis of oligonucleotides, particularly oligodeoxyribonucleotides.

Another object of this invention is to more efficiently obtain synthetic oligodeoxyribonucleotides.

The manner in which these ane other subjects are obtained will become apparent from the following description of the invention.

## SUMMARY OF THE INVENTION

According to the present invention there is provided a process for the synthesis of polynucleotides of defined sequence comprising the step of coupling, as a first moiety, a nucleotide having a masked 3'-phosphate diester group with, as a second moiety, a nucleoside or nucleotide having an available free 5'-hydroxyl group, the coupling being accomplished in the presence of a coupling agent, wherein said first moiety is employed in a substantial molar excess relative to said second moiety.

By using a substantial excess of the first moiety, the second moiety is completely or substantially completely consumed. Thus, the desired product of the coupling reaction can readily be separated from the reaction mixture since the first moiety is a charged specie.

## BRIEF DESCRIPTION OF THE FIGURES

Fig. 1 illustrates reactants useful in the present invention.

Figs. 2-4 diagram reaction schemes useful in the present invention by which di-, tri- and higher oligonucleotides can be prepared.

DESCRIPTION OF THE PREFERRED EMBODIMENTS

As previousy indicated, the present invention is an improvement upon the "triester approach" for synthesizing oligonucleotides wherein the nucleotides making up the oligonucleotide chain vary in a defined sequence. The preferred triester synthesis has involved coupling two smaller fragments, one a nucleotide having a masked but reactive 3'-phosphate diester group whereas the other is a nucleoside or nucleotide having an available free 5'-hydroxyl group, in the presence of conventional coupling agents such as 2,4,6-triisopropylbenzenesulfonyl tetrazolide in solution. Pyridine is the solvent of choice. It will be appreciated by those skilled in the art that the two reactants may themselves be oligonucleotides capable of being coupled to form a longer oligonucleotide chain.

The conventional approach has been to employ the reactants in stoichiometric, or nearly stoichiometric quantities. However, as previously pointed out, this has resulted in a reaction mixture from which it is difficult to separate the desired product. The difficulty in separation is largely a result of the fact that the reactants are not completely consumed in the reaction. As a result, the reaction mixture contains the reactive moiety having the free 5'-hydroxyl group and the desired product as well. Both of these are neutral compounds and are frequently difficult to separate.

Unexpectedly, we have found that even in solution the coupling reaction can be caused to go to completion, or substantially to completion, with respect to the moiety having the available 5'-hydroxyl group if a substantial excess of the other moiety is employed. The mixture resulting from conducting the reaction in this way contains, as the only significant products, the coupled product and the unreacted moiety having the masked 3'-phosphate diester group. Since the latter is

a charged product, it can be conveniently separated from the uncharged product of the coupling reaction.

The triester approach is most commonly used to synthesize oligonucleotides suitable for transformation to oligodeoxyribonucleic acids of defined sequence by the removal of the protective groups employed to shield various potentilally reactive sites. Accordingly, the details of the process of this invention will be explained with reference to experimental examples for preparing oligodeoxyribonucleotides. However, those skilled in the art will appreciate that the reaction can be employed to prepare oligoribonucleotides if steps are taken to protect the 2'-hydroxyl group found in oligoribonucleotides.

Referring now to Figure 1, there is shown, as formula I and formula II, mononucleotides and oligonucleotides which can be employed in the process of the present invention as the moiety having a masked 3'-phosphate diester group. In those formulae, B is selected from thymine or protected adenine, cytosine or guanine. Thymine, adenine, cytosine and guanine are, of course, the four base groups that distinguish the nucleotide fragments in a deoxyribonucleic acid (DNA). Typically, adenine and cytosine are benzoylated to protect their free amino group where as the amino group of guanine is usually protected by a isobutyryl group. Thymine, which has no free amino group, needs no such protection.

The group X in formulae I and II is a protecting group for the 5'-hydroxyl and is an organic group. Preferably, the group is one removable in a mild acid medium. Such groups include tetrahydropyrenyl, 1-methoxycyclohexyl, 4-monomethoxytrityl and 4,4'-dimethoxytrityl. The 4,4'-dimethoxytrityl is the most preferred of these and can readily be removed in mild acid medium, for example, 2% benzene sulfonic acid.

$R^1$ is a masking group for the 3'-phosphate group. Preferably, it is a group removable using a basic medium. Among suitable groups may be mentioned phenyl,

orthochlorophenyl, 2,4-dichlorophenyl and thiophenyl. A particularly preferred group is p-chlorophenyl. In formula II, n is O or an integer. Thus, formula II includes dimers and higher oligonucleotides.

Formulae III and IV of Figure 1 represent nucleosides and nucleotides, including oligonucleotides, which can be used as the reactive moiety having the available 5'-hydroxyl group. In formulae III and IV, B is as previously defined and $R^2$ is either a group for protecting the 3'-hydroxyl group which is cleavable in a basic medium or a group of the formula

```
    '
O=P-O-R3
    '
    O
    '
    R1
```

wherein $R^1$ is as previously defined and $R^3$ is a group cleavable, usually in a basic medium also. Preferably $R^3$ is a group which can be selectively removed without removing $R^1$ and $R^2$. See Itakura et al, "Improved Triester Approach For The Synthesis of Pentadecathymidylic Acid", J. Am. Chem. Soc., 97, 7327 (1975). A particularly easy group for removal is $\beta$-cyanoethylgroup and, for this reason, it is the group of choice for $R^3$. However, 1-methyl-3-ketobutyl and 2,2,2-tribromoethyl (removable using Zn) are also suitable. The group $R^2$ is preferably benzoyl but other suitable groups include acetyl, para-methoxybenzoyl, and monochloroacetyl. In Formula IV, n is 0 or an integer and, therefore, includes dimers and higher oligonucleotides.

Suitable coupling agents which can be used in the present invention are known to those skilled in the art. The previously mentioned 2,4,6-triisopropylbenzenesulfonyl tetrazolide is presently preferred. Among other suitable agents may be mentioned para-nitrobenzenesulfonyl triazolide, benzenesulfonyl triazolide,

benzenesulfonyl 4-nitroimidazolide, 2,4,6-trimethybenzenesulfphonyl tetrazolide, 2,4,6-triisopropylbenzenesulfonyl chloride and 2,4,6-trimethylbenzenesulfonyl chloride.

As has already been stated, the process of the present invention contemplates use of the first moiety having a masked 3'-phosphate diester group in a molar amount that substantially exceeds the molar amount of the second moiety having the free 5'-hydroxyl group. The amount can vary according to the nature of the reactants but, in any event, the first moiety is used in an amount that is sufficient to cause the second reactant to be substantially completely consumed. The coupling agent is employed in a molar amount that is at least about equal to the amount of first moiety and preferably is used in a molar amount that is at least twice that of the first moiety.

The relative excess of the first moiety required to bring about the consumption of the second moiety increases as the chain length of the nucleotides used in the coupling reaction increases. Therefore, while theoretically any length chain can be prepared using this process, a practical upper limit for the chain length obtained by coupling shorter fragments is one having about 21 nucleotide units. This practical upper limit is also a function of the difficulty of removing protective groups from the masked phosphate as the chain length of the oligonucleotide grows. Usually the molar ratio of the first moiety to the second is at least about 2:1. Of course, the ratio may be greater but typically need not exceed about 4:1 even when coupling a fragment containing up to about 18 nucleotide units.

The reaction time required to bring about substantially complete consumption of the second moiety can also vary depending upon the nature of the reactants and is particularly a function of the chain length of the nucleotide units being coupled. Thus, coupling to produce dimeric nucleotides can usually be complete in

60 minutes or less. By contrast, coupling of two trimer blocks can require three or more hours to go to completion. The determination of a suitable reaction time can readily be determined since progress of the reaction can be checked, for example, by using thin layer chromatography.

The greatest advantage to the present invention lies in the ease in which the reaction mixture can be resolved to yield pure or substantially pure coupled product. This advantage arises from the fact that the two components of the reaction mixture are the coupled product a neutral compound, and the unreacted first moiety which is a charged specie. When the charged specie is one that is soluble in water, it is possible to remove it from the coupled product by simple extraction using an aqueous extractant such as sodium bicarbonate solution. In the case of a reaction mixture in which the charged specie is not water soluble, separation can be achieved using other techniques, for example, short column chromatography on silica gel.

## EXAMPLE I

## SYNTESIS OF DI- AND TRINUCLEOTIDES

Figures 2 and 3 illustrate, respectively, the use of the process of this invention to form di-and trideoxyribonucleotides. The coupling reagent employed was the preferred triisopropylbenzenesulfonyl tetrazolide (TPSTe). The protecting group for the 5'-hydroxyl was the 4,4'-dimethoxytrityl group (DMTr). In the reactions performed, $R_2$ was either benzoyl (group b) or the group

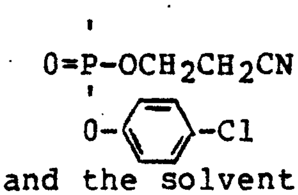

(group a) and the solvent used for the coupling reaction was pyridine.

With reference to Figure 2, using an excess of 1 (2 mmole), the coupling reaction with 2 (1 mmole) went substantially to completion in about 60 minutes with the aid of the coupling reagent TPSTe (4 mmole) to form coupled product 3 in a mixture containing unreacted 1. The reaction mixture was then treated with 2% benzene sulfonic acid solution for 10 minutes at 0°C to remove the 4,4'-dimethoxytrityl groups of 1 and 3 to produce 4 and 5. By reason of removal of the protective group, compound 4 is soluble in aqueous sodium bicarbonate solution. Thus, 4 and 5 can be separated by a simple extraction of 4 from chloroform using the aqueous sodium bicarbonate solution. The 5'-hydroxyl dinucleotide 5 in choloroform solution is, then precipitated from ether to give a homogeneous colorless product as indicated by thin layer chromatography on silica gel. It will be appreciated by those skilled in the art, that where it is desired to retain the 4,4'-dimethoxytrityl group on the coupled product, the reactant 1 can be separated from coupled product 3 by means of a column chromatography using, for example, silica gel as a solid phase.

Turning now to Figure 3, the fully protected trimer block 6 was prepared from dinucleotide 5 (1 mmole), and compound 1 (2 mmole) in the presence of TPSTe (4 mmole). Separation from 1 was accomplished by means of short column chromatography on silica gel. After separation, the product was shown to be homogeneous by thin layer chromatography on silica gel and, after removal of the protecting groups, by thin layer chromatography on cellulose. The yields of fully protected trimers 6 based upon the starting materials compound 2 is shown in Table 1 below. These modifications of the triester approach using an excess of the 3'-phosphodiester component 1 let to an increase in overall yield as well as a decrease in the working time by at least a factor of 2 as compared to the conventional triester approach.

TABLE 1

Yields of Fully Protected Trimers (VI)

| Sequence[1] | Yield | Sequence | Yield |
|---|---|---|---|
| TTT(b) | 81% | ATG(b) | 69% |
| TTT(a) | 75% | GCC(a) | 61% |
| GGA(a) | 41% | CCA(a) | 72% |
| AGA(a) | 49% | CAA(a) | 72% |
| ATC(a) | 71% | TTA(a) | 71% |
| CCT(a) | 61% | CAT(a) | 52% |
| ACA(a) | 63% | CCC(a) | 73% |
| ACC(a) | 65% | AAC(a) | 59% |
| CGT(b) | 51% | GAT(a) | 60% |

1 The identity of the nucleotide in each sequence is indicated by the capital letter representative of the substituent B. The letter in parenthesis identifies the group $R^2$ as indicated above.

The ability to synthesize trinucleotide blocks in high yield by the process of this invention is particularly significant because proper positioning of trimer blocks in DNA codes it to stimulate the production of a specific amino acid as part of a polypeptide chain. For example, the block TTT stimulates the production of phenylalanine. Similarly, the block GGA stimulates the production of glycine. Accordingly, the trimer blocks derived by the process of this invention can be serially coupled to form an oligonucleotide which, after removal of protective groups, forms a DNA molecule that, if properly inserted in plasmid DNA, will stimulate formation of a polypeptide chain having a desired sequence of amino acids. The coupling of trimer blocks to form longer oligonucleotides having a defined sequence is described in Example II.

## EXAMPLE II

## SYNTHESIS OF OLIGODEOXYRIBONUCLEOTIDES

With reference now to Figure 4, the synthesis of oligodeoxyribonucleotides by the process of the present invention is outlined. Thus, the coupling of an excess of the 3'-phosphodiester trimer block X (n = 1, 1.5 mmole) with the 5'-hydroxyl trimer block Y (m = 1, 0.5 mmole) in the presence of TPSTe, 3 mmole, in pyridine as a solvent, could be forced substantially to completion in about 3 hours.

Attempts to remove the unreacted starting material X from the product Z, a hexamer, by first shaking the reaction mixture with an ion exchange resin such as Dowex 1-X8 and DEAE-cellulose in various aqueous organic solvents followed by filtration were not successful since complete removal of the reactant X could not be accomplished. However, the charged component X could be removed by passing the reaction mixture through a very short (3 cm long) and wide (7 cm diameter) silica gel column.

The column is first washed with chloroform (100 ml.) to elute side products and the coupling reagent, and then with a more polar elutant $CHCl_3$-MEOH (95:5 V/V, 250 ml.) to elute almost all of the fully protected hexamer Z. The charged component X remained on the column.

The fraction containing the hexamer was evaporated to produce a colorless solid which was treated with 2% benzene sulfonic acid solution in $CHCl_3$-MEOH (7:3 V/V) to remove the 5'-hydroxyl protecting group to yield compound Y (m = 4). This hexamer could be precipitated from ether and used in a subsequent coupling reaction without further purification.

Repetition of this cycle 4 times i.e., using X wherein n = 1 with Y wherein M = 1, 4, 7, and 10

successively, yielded the fully protected pentadecamer Z (n = 1, m = 12) in yields of about 40-50% overall from the 5'-hydroxyl trimer block Y (m =1) in only about 4 days. The final product, i.e., the pentadecamer, was purified by high pressure liquid chromatography after removal of all the protecting groups. By this approach the oligonucleotides d (Tp)$_{14}$T and d ApCpApCpCpCpApAp GpApCpCpCpGpT, and d TpTpTpGpTpCpApApTpCpApGpGpApC were synthesized.

Confirmatory evidence for the structure of the pentadecamers was obtained by homochromatography on thin layer DEAE-cellulose and by gel electrophoresis on a 20% acrylamide slab after incubation of the pentadecamers with [$\gamma$-$^{32}$P]-adenosine 5'-triphosphate in the presence of T4-phosphokinase. One major labeled product was obtained from each substrate.

Furthermore, the labeled products were partially digested with venom phosphodiesterase and the digests were submitted to two dimensional homochromatography which showed the expected sequence pattern of the two pentadecamers. The results obtained in this example demonstrate that coupling reactions between a short oligomer block (the trimer X, n = 1) and longer oligomer blocks go substantially to completion using an excess of the trimer block and that higher oligomers can be synthesized quickly and without the necessity of performing extensive purification steps on intermediate products. By appropriate selection of the trimer blocks employed in successive coupling reactions, oligonucleotides containing codons for specific amino acids in a desired sequence can be rapidly prepared.

## CLAIMS

1. A process for the synthesis of polynucleotides of defined sequence comprising the step of coupling, as a first moiety, a nucleotide having a masked 3'-phosphate diester group with, as a second moiety, a nucleoside or nucleotide having an available free 5'-hydroxyl group, the coupling being accomplished in the presence of a coupling agent, characterised in that said first moiety is employed in a substantial molar excess relative to said second moiety.

2. A process according to claim 1 wherein the second moiety is substantially completely consumed in the coupling reaction.

3. A process according to claim 2 wherein the molar ratio of said first moiety to said second moiety is at least about 2:1.

4. A process according to any one of the preceding claims wherein the coupling reaction is done in solution.

5. A process according to claim 4 wherein the coupling reaction is conducted in pyridine as the solvent.

6. A process according to any one of the preceding claims wherein the coupling agent is triisopropylbenzenesulfonyl tetrazolide.

7. A process according to any one of the preceding claims wherein said first moiety is of the formula:

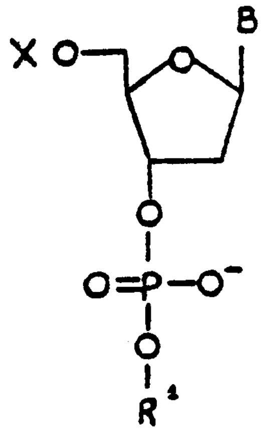

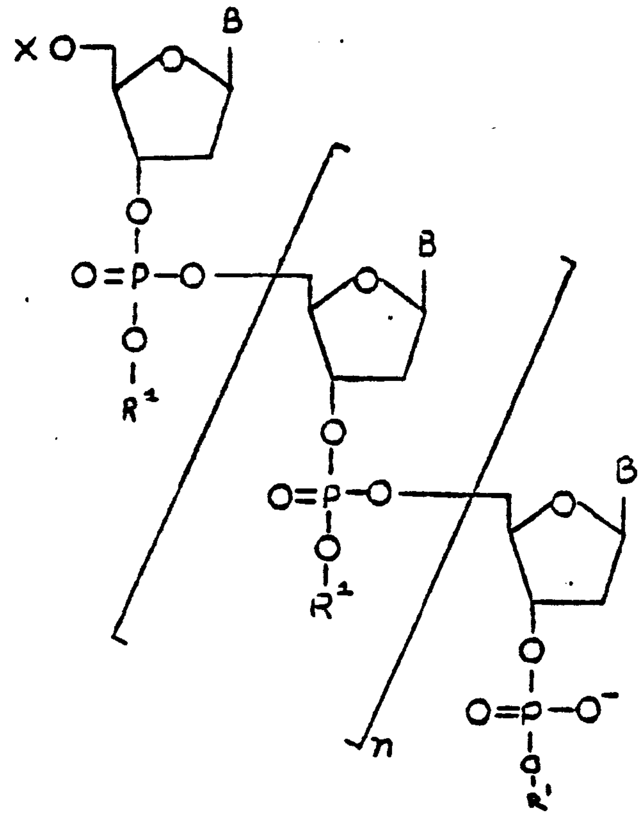

said second moiety is of the formula:

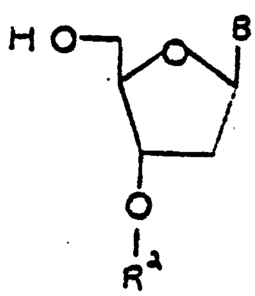

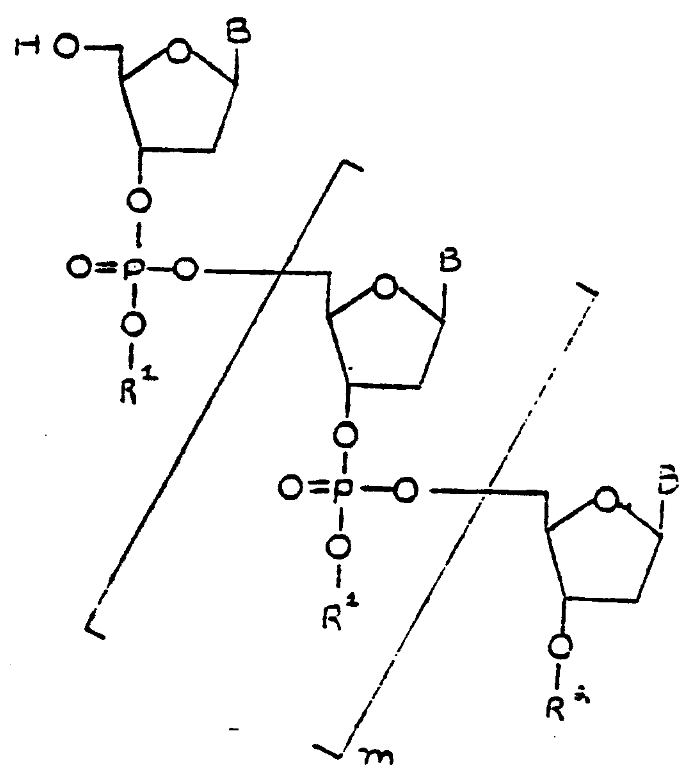

and wherein B can be the same or different and is thymine, protected adenine, cytosine, or guanine; wherein X is an organic group labile in acid medium; wherein $R^1$ is an organic group labile in a basic medium; wherein $R^2$ is either an organic group labile in a basic medium or a goup of the structure

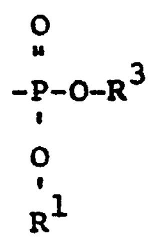

wherein $R^1$ is as defined above and $R^3$ is a group labile in a basic medium under conditions wherein said $R^1$ and said $R^2$ groups are not removed; and wherein *n* and *m* are independently O or an integer.

8. A process according to claim 7 wherein X, is tetrahydrophyrenyl, 1-methoxycyclohexyl, 4-monomethoxytrityl or 4,4'-dimethoxytrityl.

9. A process according to claim 8 wherein X is 4,4'-dimethoxytrityl.

10. A process according to any one of claims 7 to 10 wherein each $R^1$ which may be the same or different, is phenyl, *o*-chlorophenyl, 2,4-dichlorophenyl, *p*-nitrophenyl, 2,2,2-trichloroethyl or *p*-chlorophenyl.

11. A process according to claim 10 wherein at least one $R^1$ is *p*-chlorophenyl.

12. A process according to any one of claims 7 to 11 wherein $R^2$ is a group labile in a basic medium and is acetyl, p-methoxybenzoyl, monochloroacetyl, 2,2,2-tribromoethyl,

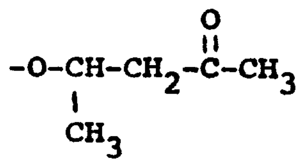

or

benzoyl.

13. A process according to claim 12 wherein $R^2$ is benzoyl.

14. A process according to any one of claims 7 to 11 wherein $R^2$ is

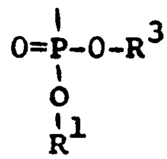

and $R^3$ is $-O-CH_2CH_2CN$.

15. A process according to any one of claims 7 to 11 wherein $R^2$ is

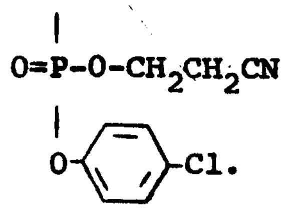

16. A process according to any one of claims 7 to 15 wherein the coupling agent is triisopropylbenzenesulfonyl tetrazolide, X is 4,4'-dimethoxytrityl, $R^1$ is p-chlorophenyl, n is 1 and m is 1, 4, 7 or 10.

17. A process for the preparation of nucleotides comprising the steps:

a) coupling in the presence of a coupling agent first and second moieties of the formulae:

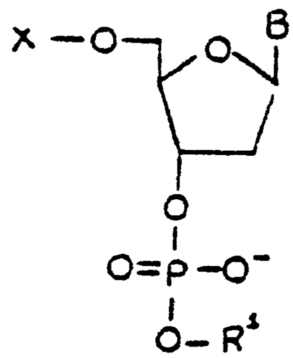

first moiety

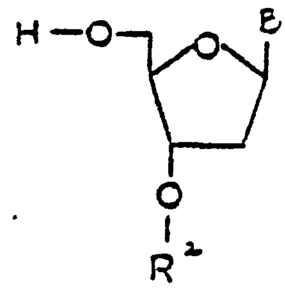

second moiety

wherein X is an organic group labile in an acid medium, $R^1$ is a group labile in a basic medium and $R^2$ is a group labile in a basic medium or the group

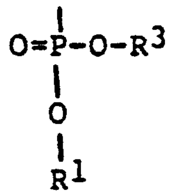

wherein $R^3$ is a group labile in a basic medium

under conditions wherein $R^1$ and $R^2$ are not removed and each B which may be the same or different, is thymine, protected adenine, cytosine or guanine, said first moiety being employed in a substantial molar excess relative to said second moiety, said coupling reaction being maintained for a time sufficient to substantially consume said second moiety and produce a coupled product of the formula

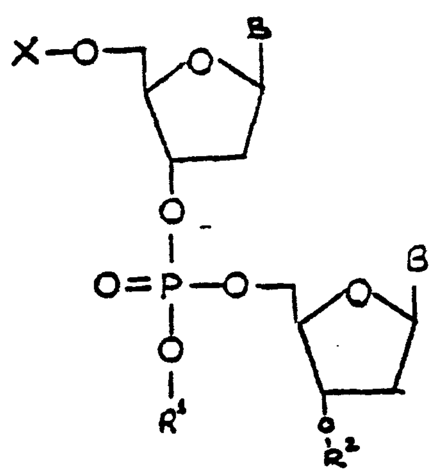

b) removing the group X from the coupled product and the unreacted first moiety by contacting the reaction mixture with an acid medium to produce a mixture comprising the charged specie

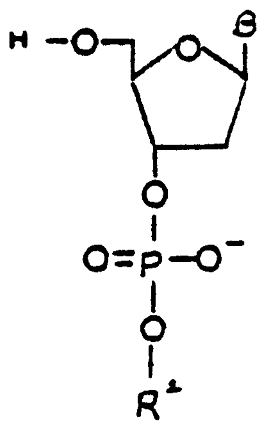

and the dinucleotide

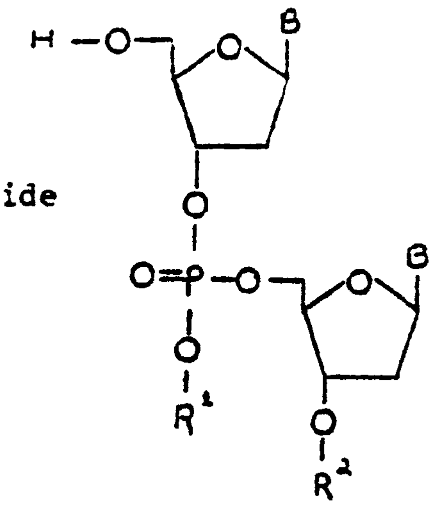

and;

c) separating the dinucleotide of step b) from the mixture.

18. A process according to Claim 17 wherein the separation is by extraction with aqueous bicarbonate to remove the charged specie.

19. A process according to Claim 17 or 18 comprising the further steps of coupling said first moiety and said dinucleotide, wherein said first moiety is employed in a substantial molar excess relative to said dinucleotide, in the presence of a coupling agent for a time sufficient to substantially completely consume said dinucleotide to form a mixture comprising unreacted first moiety and a trinucleotide of the formula

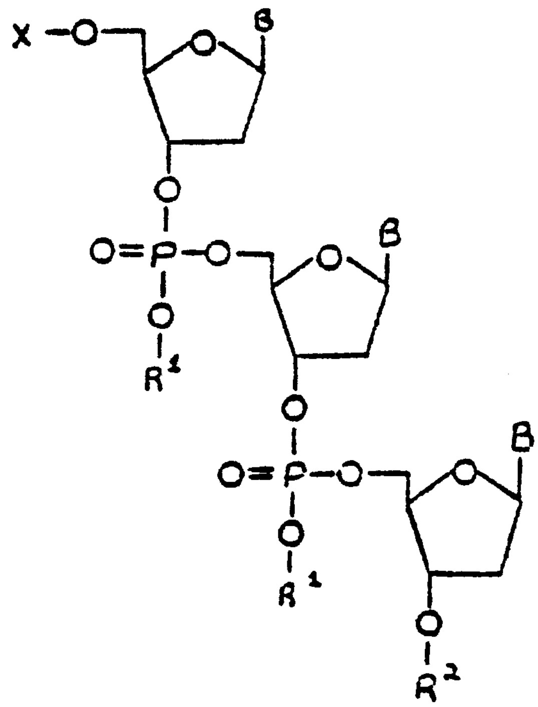

and separating said trinucleotide from said mixture.

20. A process according to Claim 19 wherein the unreacted first moiety and the trinucleotide are separated using column chromatography.

21. A process according to any one of claims 17 to 20 wherein the coupling agent is triisopropylbenzenesulfonyl tetrazolide, X is 4,4'-dimethoxytrityl, $R^1$ is p-chlorophenyl and $R^2$ is benzoyl or the group

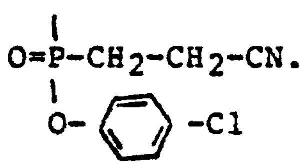

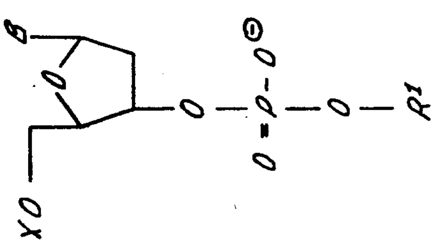

I

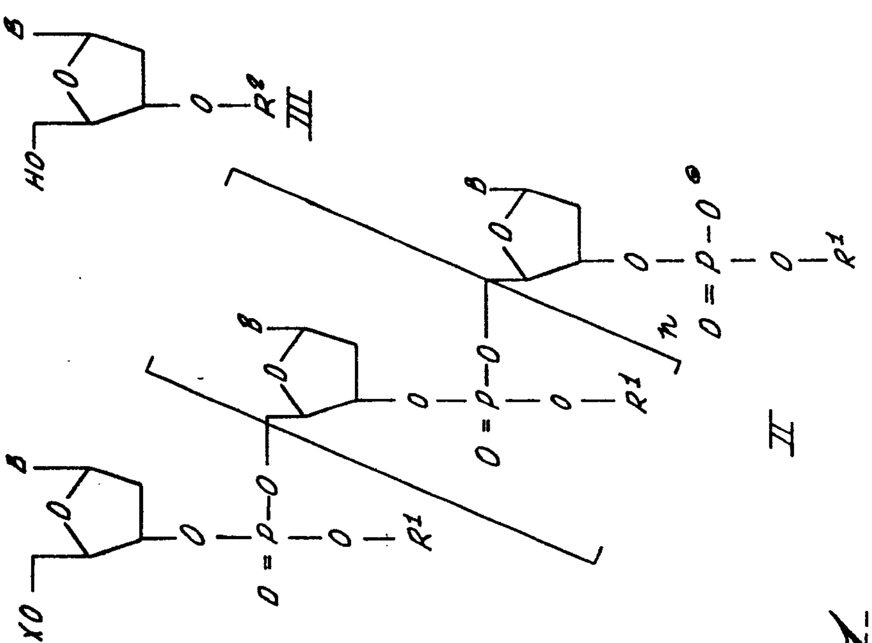

II

III

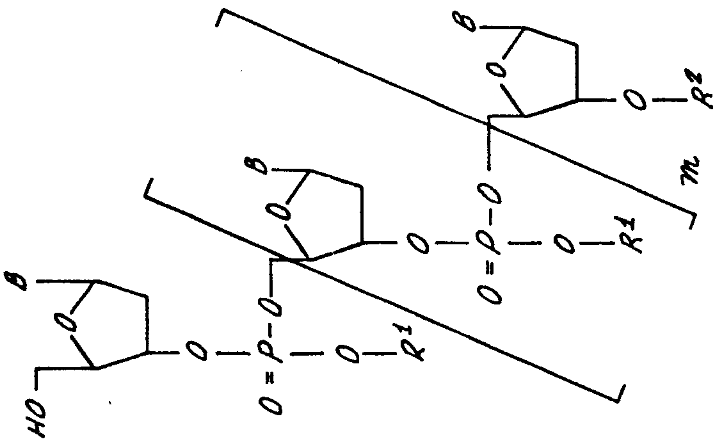

IV

FIG. 1

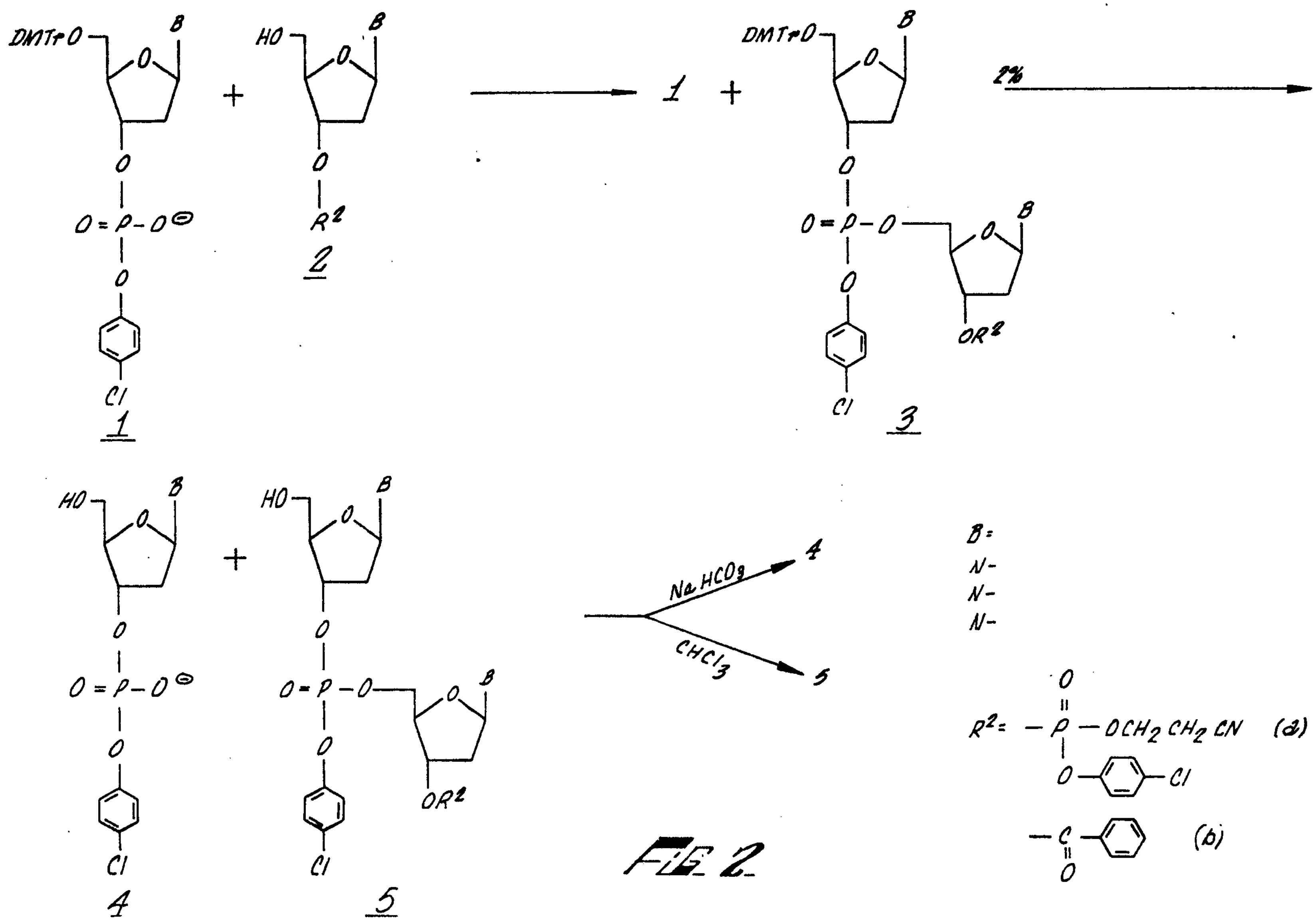


1 + 2 → 1 + 3

4 + 5

B=
N-
N-
N-

$R^2$ = (a)

(b)

FIG. 2

0002322

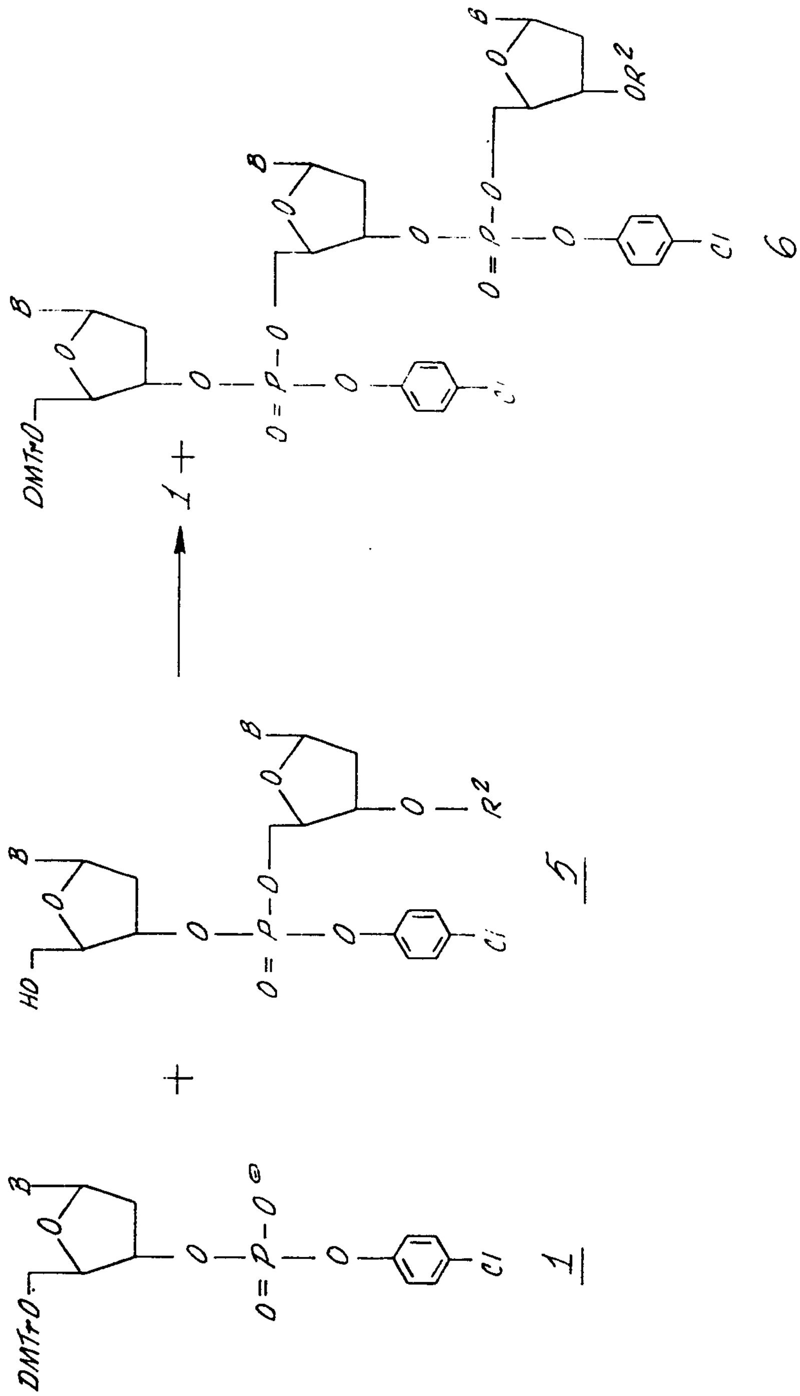


1

5

6

Fig. 3

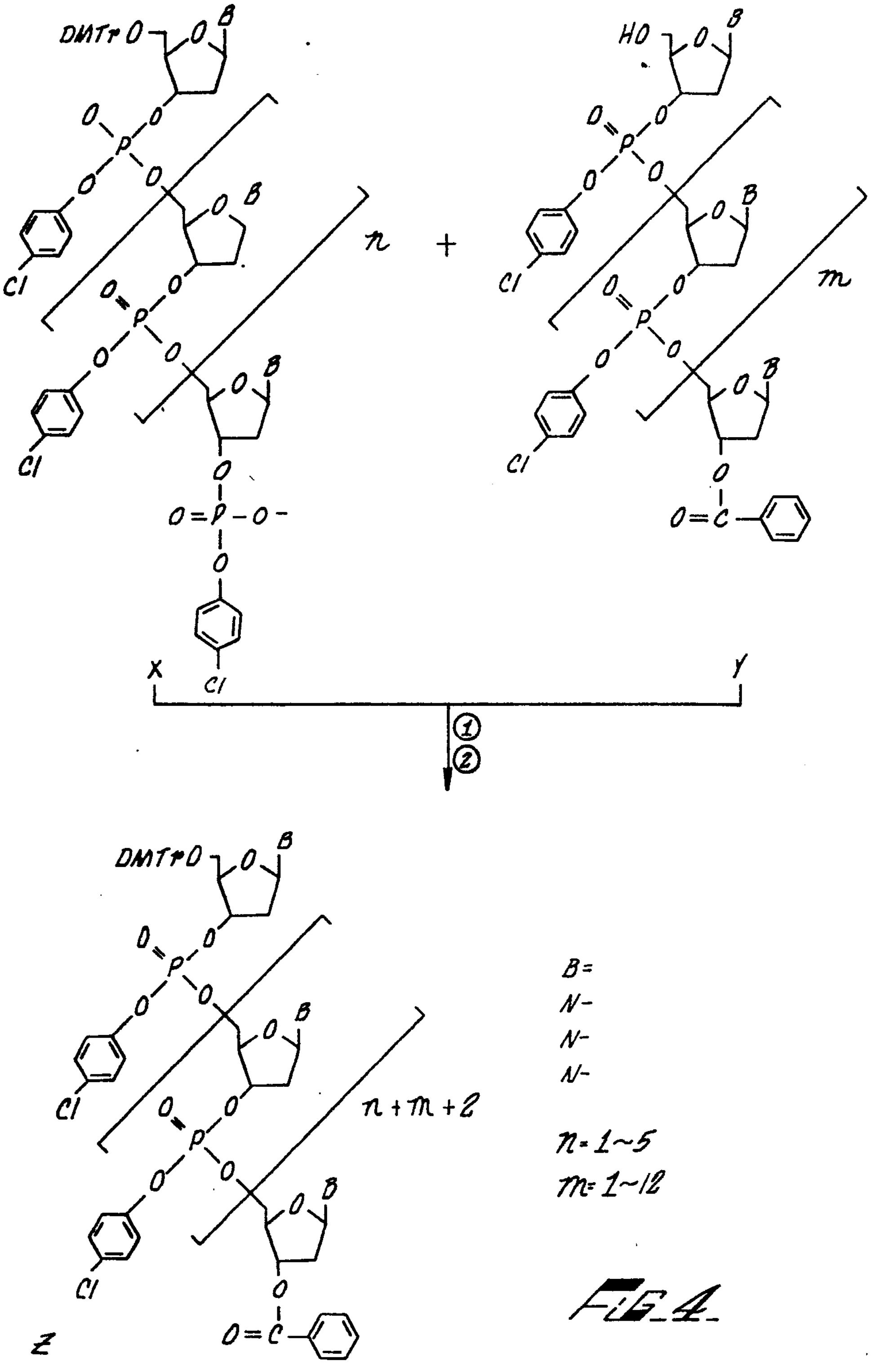

X

Y

Z

B=
N-
N-
N-

n = 1~5
m = 1~12

FIG. 4.